# EUROPEAN PATENT APPLICATION

(11) **EP 4 410 795 A1**
(43) Date of publication of application: **07.08.2024**
(21) Application number: 22876952.7
(22) Date of filing: 30.09.2022
(51) Int. Cl.: C07D 495/04, A61K 31/5377

(54) **PIKFYVE KINASE INHIBITOR**

(30) Priority: 30.09.2021 KR 20210129839
(71) Applicant: Hanmi Pharm. Co., Ltd., Hwaseong-si, Gyeonggi-do 18536 (KR)
(72) Inventor: SUH, Kwee Hyun, Hwaseong-si, Gyeonggi-do 18469 (KR); JANG, Sun Young, Hwaseong-si, Gyeonggi-do 18469 (KR); JUN, Seung Ah, Hwaseong-si, Gyeonggi-do 18469 (KR); KIM, Ye Lim, Hwaseong-si, Gyeonggi-do 18469 (KR); CHUNG, Shin Hyuck, Hwaseong-si, Gyeonggi-do 18469 (KR)
(74) Representative: Brevalex
(86) International application number: PCT/KR2022/014781
(87) International publication number: WO 2023/055181

(57) **Abstract**

The present disclosure relates to a thieno[3,2-d]pyrimidine derivative compound which is useful as a PlKfyve kinase inhibitor, a pharmaceutically acceptable salt thereof, a preparation method thereof, and an intermediate thereof.

## Description

### Technical Field

The present disclosure relates to a thieno[3,2-*d*]pyrimidine derivative compound which is useful as a PlKfyve kinase inhibitor, a pharmaceutically acceptable salt thereof, a preparation method thereof, and an intermediate thereof.

### Background Art

Phosphorylated derivatives of phophatidylinositol (PI) regulate cytoskeletal functions, membrane trafficking, and receptor signaling by recruiting protein complexes to cells and endosomal membranes. The PlKfyve signaling pathway has been reported to regulate a number of biological processes, mostly through well-documented roles in endosomal trafficking. PlKfyve refers to a FYVE finger-containing phosphoinositide kinase, which is an enzyme encoded by the PIKFYVE gene in human, and this enzyme is also known as phosphatidylinositol-3-phosphate 5-kinase type III or PIPKIII. PlKfyve is to phophorylate phosphatidylinositol 3-phosphate (also referred to as 'Ptdlns(3)P' or 'PI(3)P') to phosphatidylinositol 3,5-bisphosphate (also referred to as 'PtdIns(3,5)P₂' or 'PI(3,5)P₂'), and PtdIns(3,5)P₂ thus produced is essential for maintaining late endocytosis integrity. A decrease in the PlKfyve enzymatic activity may cause endosome expansion and cytoplasm vacuolation due to a decrease in PI(3,5)P₂ synthesis. In addition, PlKfyve is involved in the production of phosphatidylinositol 5-phosphate (PI5P), and regulates celllar endisomal events (division and fusion) that maintain proper performance of transport pathways and intracellular membrane homeostasis.

### Disclosure

### Technical Problem

The present disclosure provides a thieno[3,2-d]pyrimidine derivative compound which is useful as a PlKfyve kinase inhibitor, a pharmaceutically acceptable salt thereof, a preparation method thereof, and an intermediate thereof.

### Technical Solution

### Compound for treatment

An aspect provides a compound of Formula (1) or a pharmaceutically acceptable salt thereof. 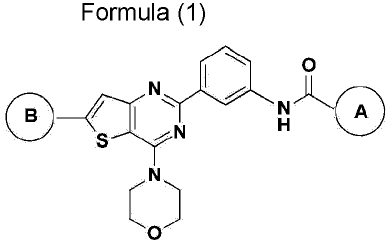 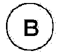
may be 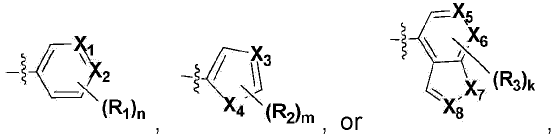 wherein X₁, X₂, X₃, X₅, X₆, or X₃ may each independently be CH or a nitrogen atom, and
X₄ and X₇ may each independently be CH₂, an oxygen atom, or NH,
in each case, R₁, R₂, and R₃ may each independently be one selected from a halogen, an amino group, -NH(C₁-C₃alkyl), -N(C₁-C₃alkyl)₂, C₁-C₆alkyl, C₂-C₆alkenyl, and C₁-C₆alkoxy,
n may be an integer of 0 to 4,
m may be an integer from 0 to 2,
k may be an integer of 0 to 4, 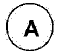
may be 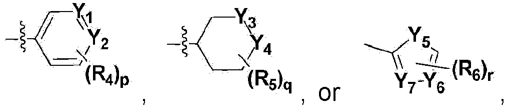 wherein Y₁, Y₂, Y₆, and Y₇ may each independently be CH or a nitrogen atom, and
Y₃, Y₄, and Y₅ may each independently be CH₂, an oxygen atom, NH, or a sulfur atom,
in each case, R₄ and R₆ may each independently be one selected from hydrogen, a halogen, an amino group, C₁-C₆alkyl, C₂-C₆alkenyl, and C₁-C₆alkoxy,
R₅ may be one selected from hydrogen, -T-OH, -T-CHO, -T-(C₁-C₄alkyl), and -T-(C₁-C₄alkoxy), wherein -T- may be absent or may be -C(=O), C₁-C₄alkylene or C₂-C₄alkenylene,
p may be an integer of 0 to 4,
q may be an integer of 0 to 4, and
r may be an integer from 0 to 2.

In an embodiment, 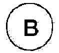 may be one of 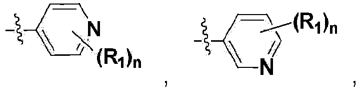 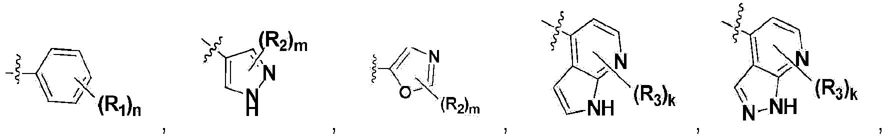 and

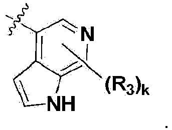

In an embodiment, 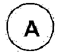 may be one of

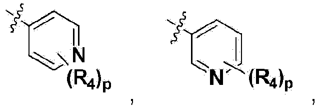

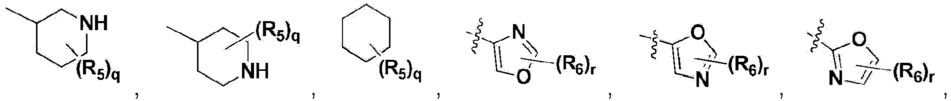

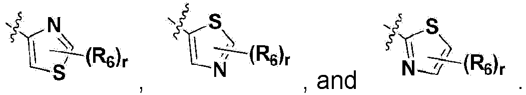

In an embodiment, R₁ may be a halogen or an amino group.

In an embodiment, R₅ may be one of hydroxy, -(C₁-C₄alkylene)-OH, -(C₁-C₄alkylene)-(C₁-C₄alkoxy), -C(=O)H, -C(=O)(C₁-C₄alkyl), -C(=O)-(C₁-C₄alkoxy), and - (C₁-C₄alkylene)-CHO.

In an embodiment, R₆ may be one of methyl, ethyl, and propyl.

The term "halogen" as used in the present specification may include fluorine, chlorine, bromine, or iodine, unless otherwise specified.

The term "alkyl" as used in the present specification refers to a saturated monovalent hydrocarbon radical. The term "alkenyl" as used in the present specification refers to a monovalent hydrocarbon radical containing at least one carbon-carbon double bond, wherein each double bond may have an E- configuration or a Z-type configuration.

The term "alkoxy" as used in the present specification refers to a straight-chain or branched hydrocarbon residue linked by oxygen. The term "alkylene" as used in the present specification refers to a divalent straight-chain or branched hydrocarbon group having (-CH₂-)ₙ.

Such alkyl, alkenyl, alkoxy, and alkylene groups may each be a linear type, i.e., a straight-chain, or a brached type with side chains.

In the present specification, -(C₁-C₄alkylene)-OH is also referred to as a hydroxy(C₁-C₄)alkyl group, and may be, for example, a hydroxymethyl group, a hydroxyethyl group, a hydroxypropyl group, or a hydroxybutyl group.

In the present specification, a -(C₁-C₄alkylene)-(C₁-C₄alkoxy) group is also referred to as a (C₁-C₄)alkoxy(C₁-C₄)alkyl group, and may be, for example, a methoxymethyl group, a methoxyethyl group, a methoxypropyl group, an ethoxypropyl group, or a propoxymethyl group.

In the present specification, -C(=O)H is also referred to as a formyl group (-CHO).

In the present specification, -C(=O)(C₁-C₄alkyl) is also referred to as a (C₁-C₄)acyl group, and may be, for example, an acetyl group (-COCH₃), a propionyl group (-COCH₂CH₃), or a butyryl group (-COCH₂CH₂CH₃).

In the present specification, -C(=O)-(C₁-C₄alkoxy) is also referred to as a (C₁-C₄alkoxy)carbonyl group, and may be, for example, a methoxycarbonyl group, an ethoxycarbonyl group, a propoxycarbonyl group, an isopropoxycarbonyl group, a butoxycarbonyl group, an isobutoxycarbonyl group, or a tert-butoxycarbonyl group.

In the present specification, -(C₁-C₄alkylene)-CHO is also referred to as a formyl(C₁-C₄)alkyl group, and may be, for example, a formylmethyl group, a formylethyl group, a formylpropyl group, or a formylisopropyl group.

In addition, preferable examples of the compound of Formula (1) according to the present disclosure are as follows, but are not limited thereto:
*N*-(3-(4-morpholino-6-(pyridin-4-yl)thieno[3,2-*d*]pyrimidin-2-yl)phenyl)isonicotinamide
*N*-(3-(4-morpholino-6-(pyridin-3-yl)thieno[3,2-*d*]pyrimidin-2-yl)phenyl)isonicotinamide
*N*-(3-(4-morpholino-6-(oxazol-5-yl)thieno[3,2-*d*]pyrimidin-2-yl)phenyl)isonicotinamide
*N*-(3-(4-morpholino-6-(pyridin-4-yl)thieno[3,2-*d*]pyrimidin-2-yl)phenyl)nicotinamide
*N*-(3-(4-morpholino-6-(pyridin-3-yl)thieno[3,2-*d*]pyrimidin-2-yl)phenyl)nicotinamide
*N*-(3-(6-(4-aminophenyl)-4-morpholinothieno[3,2-*d*]pyrimidin-2-yl)phenyl)nicotinamide hydrochloride
*N*-(3-(4-morpholino-6-(oxazol-5-yl)thieno[3,2-*d*]pyrimidin-2-yl)phenyl)nicotinamide
*N*-(3-(4-morpholino-6-(1H-pyrazole-4-yl)thieno[3,2-*d*]pyrimidin-2-yl)phenyl)isonicotinamide
*N*-(3-(4-morpholino-6-(1H-pyrrolo[2,3-*b*]pyridin-4-yl)thieno[3,2-*d*]pyrimidin-2-yl)phenyl)nicotinamide
*N*-(3-(4-morpholino-6-(1H-pyrazolo[3,4-*b*]pyridin-4-yl)thieno[3,2-*d*]pyrimidin-2-yl)phenyl)nicotinamide
*N*-(3-(4-morpholino-6-(1H-pyrrolo[2,3-c]pyridin-4-yl)thieno[3,2-*d*]pyrimidin-2-yl)phenyl)nicotinamide
*N*-(3-(4-morpholino-6-(pyridin-3-yl)thieno[3,2-*d*]pyrimidin-2-yl)phenyl)oxazole-4-carboxamide
*N*-(3-(4-morpholino-6-(pyridin-3-yl)thieno[3,2-*d*]pyrimidin-2-yl)phenyl)oxazole-5-carboxamide
*N*-(3-(4-morpholino-6-(pyridin-3-yl)thieno[3,2-*d*]pyrimidin-2-yl)phenyl)oxazole-2-carboxamide
2-methyl-*N*-(3-(4-morpholino-6-(pyridin-3-yl)thieno[3,2-*d*]pyrimidin-2-yl)phenyl)oxazole-5-carboxamide
4-methyl-*N*-(3-(4-morpholino-6-(pyridin-3-yl)thieno[3,2-*d*]pyrimidin-2-yl)phenyl)oxazole-5-carboxamide
2,4-dimethyl-*N*-(3-(4-morpholino-6-(pyridin-3-yl)thieno[3,2-*d*]pyrimidin-2-yl)phenyl)oxazole-5-carboxamide
*N*-(3-(4-morpholino-6-(pyridin-3-yl)thieno[3,2-*d*]pyrimidin-2-yl)phenyl)thiazole-4-carboxamide
*N*-(3-(4-morpholino-6-(pyridin-3-yl)thieno[3,2-*d*]pyrimidin-2-yl)phenyl)thiazole-5-carboxamide
*N*-(3-(4-morpholino-6-(pyridin-3-yl)thieno[3,2-*d*]pyrimidin-2-yl)phenyl)thiazole-2-carboxamide
2-methyl-*N*-(3-(4-morpholino-6-(pyridin-3-yl)thieno[3,2-*d*]pyrimidin-2-yl)phenyl)thiazole-5-carboxamide
4-methyl-*N*-(3-(4-morpholino-6-(pyridin-3-yl)thieno[3,2-*d*]pyrimidin-2-yl)phenyl)thiazole-5-carboxamide
2,4-dimethyl-*N*-(3-(4-morpholino-6-(pyridin-3-yl)thieno[3,2-*d*]pyrimidin-2-yl)phenyl)thiazole-5-carboxamide
*N*-(3-(4-morpholino-6-(pyridin-3-yl)thieno[3,2-*d*]pyrimidin-2-yl)phenyl)piperidine-3-carboxamide
*N*-(3-(4-morpholino-6-(pyridin-3-yl)thieno[3,2-*d*]pyrimidin-2-yl)phenyl)piperidine-4-carboxamide
1-(2-hydroxyethyl)-*N*-(3-(4-morpholino-6-(pyridin-3-yl)thieno[3,2-*d*]pyrimidin-2-yl)phenyl)piperidine-4-carboxamide
1-(2-methoxyethyl)-*N*-(3-(4-morpholino-6-(pyridin-3-yl)thieno[3,2-*d*]pyrimidin-2-yl)phenyl)piperidine-4-carboxamide
1-formyl-*N*-(3-(4-morpholino-6-(pyridin-3-yl)thieno[3,2-*d*]pyrimidin-2-yl)phenyl)piperidine-4-carboxamide
1-acetyl-*N*-(3-(4-morpholino-6-(pyridin-3-yl)thieno[3,2-*d*]pyrimidin-2-yl)phenyl)piperidine-4-carboxamide
methyl 4-((3-(4-morpholino-6-(pyridin-3-yl)thieno[3,2-*d*]pyrimidin-2-yl)phenyl)carbamoyl)piperidine-1-carboxylate
ethyl 4-((3-(4-morpholino-6-(pyridin-3-yl)thieno[3,2-*d*]pyrimidin-2-yl)phenyl)carbamoyl)piperidine-1-carboxylate
(1s,4s)-4-hydroxy-*N*-(3-(4-morpholino-6-(pyridin-3-yl)thieno[3,2-*d*]pyrimidin-2-yl)phenyl)cyclohexane-1-carboxamide
*N*-(3-(4-morpholino-6-(oxazol-5-yl)thieno[3,2-*d*]pyrimidin-2-yl)phenyl)oxazole-4-carboxamide

### Manufacturing method

The compound of Formula (1) or a pharmaceutically acceptable salt thereof may be prepared by using known organic synthesis techniques, or may be synthesized according to any of a number of available pathways. Those skilled in the art know how to select and practice appropriate synthetic protocols, and recognize that a broad repertoire of synthetic organic reactions can potentially be used to synthesize the compounds provided in the present specification.

Reactions to prepare the compounds provided in the present specification may be carried out in suitable solvents that can be readily selected by those skilled in the art of organic synthesis. Suitable solvents may be substantially non-reactive with starting materials (reactants), intermediates, or products at the temperature at which reactions are carried out, such as a temperature that can range from the freezing temperature of the solvent to the boiling temperature of the solvent. A given reaction may be carried out in one solvent or in a mixture of more than one solvent. Depending on particular reaction steps, suitable solvents for the particular reaction processes may be selected by those skilled in the art.

### Pharmaceutical composition

The present application also provides: an effective amount of the compound of Formula (1) disclosed in the present specification or a pharmaceutically acceptable salt thereof; and a pharmaceutical composition including a pharmaceutically acceptable carrier.

Regarding the pharmaceutical composition or dosage forms, one of the compounds and therapeutic agents described in the present specification may be contained in an amount in a range of 0.005 % to 100 %, as well as a suitable pharmaceutical excipient in the remaining amount.

### Routh of administration and form of administration

The pharmaceutical composition of the present application may include substances suitable for any acceptable routh of administration.

### Mode for Invention

All technical terms as used in the present disclosure have the same meaning as commonly understood by those of ordinary skill in the relevant art, unless otherwise defined. Preferable methods or samples are described in the present specification, but similar or equivalent ones are also within the scope of the present specification. Although not explicitly stated, numerical values described in the present specification are considered to include the meaning of "about". The term "about" as used in the present specification refers to a value within 5 % of a given value or range, preferably within 1 % to 2 %. In the present specification, a numerical range expressed with the term "to" includes a numerical range including numerical values written before and after the term "to" as a lower limit and an upper limits, respectively.

Hereinafter, the present disclosure will be described in more detail through the following Examples and Experimental Examples. However, these Examples and Experimental Examples are only intended to aid understanding of the present disclosure, and the scope of the present disclosure is not limited thereto in any way.

### Example 1: Preparation of N-(3-(4-morpholino-6-(pyridin-4-yl)thieno[3,2-d]pyrimidin-2-yl)phenyl)isonicotinamide

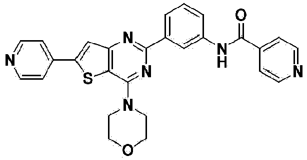

### Process 1) Preparation of 4-(2-chloro[3,2-d]pyrimidin-4-yl)morpholin

100 g of (490 mmoml)2,4-dichlorothieno[3,2-d]pyrimidine and 92.5 mL (1070 mmoml) of morpholin were diluted in 2 L of methanol, and stirred at room temperature for 2 hours. After completion of the reaction, the produced solid was filtered. The solid thus obtained was then washed, filtered under reduced pressure, and dried under reduced pressure, so as to obtain 122.8 g (yield : 98 %) of the title compound.

¹H-NMR (300MHz, DMSO-*d*₆) *δ* 8.32(d, 1H), 7.42(d, 1H), 3.94~3.91 (m, 4H), 3.78~3.75 (m, 4H).

MS (ESI⁺) *m*/*z* 204.93 [M+H]⁺.

### Process 2) Preparation of 4-(2-chloro-6-iodothieno[3,2-d]pyrimidin-4-yl)morpholin

60 g (234.6 mmol) of the compound prepared in Process 1) was dissolved in 600 mL of tetrahydrofuran, and 150 mL (370.7 mmol) of normal-butyllithium (2.5 M in hexane) was added dropwise thereto at -78 °C for 30 minutes, followed by stirring at - 40 °C for 2 hours. Next, 94.2 g (370.7 mmol) of iodine was dissolved in 600 mL of tetrahydrofuran, and this solution was added dropwise to the reaction mixture at -78 °C, followed by stirring for 1 hour. After raising the reaction temperature to room temperature, the resulting reaction mixture was washed with a saturated aqueous solution of sodium bicarbonate, and an extraction process was perofrmed thereon by using ethyl acetate. The organic layer thus separated was dried with anhydrous sodium sulfate and filtered under reduced pressure, and the filtrate was distilled under reduced pressure. The solid thus obtained was washed with diethyl ether, so as to obtain 72 g (yield: 80 %) of the title compound.

¹H-NMR (300MHz, DMSO-*d*₆) δ 7.4 (s, 1H), 3.84-3.83 (m, 4H), 3.76-3.74 (m, 4H).

MS (ESI⁺) *m*/*z* 381.92 [M+H]⁺.

### Process 3) Preparation of 4-(2-chloro-6-(pyridin-4-yl)thieno[3,2-d]pyrimidin-4-yl)morpholin

120 g (314.4 mmol) of the compound prepared in Process 2), 46.4 g (377.2 mmol) of 4-pyridine boronic acid, 1,1'-bis(diphenylphosphino)ferrocene]dichloropalladium(II), 12.8 mg (15.72 mmol) of dichloromethane composite, and 133.2 g (1257.6 mmol) of sodium carbonate were diluted in 3 L of a mixed solution containing diethylene glycol dimethyl ether and water (at a volume ratio of 4:1), and then stirred at 90 °C for 3 hours. After completion of the reaction, the resulting reaction mixture was cooled to room temperature and distilled under reduced pressure. The resulting residue was washed with water and filtered under reduced presure. The solid thus obtained was filtered through a filter filled with Celite (contianing dichloromethane and methanol at a volume ratio of 9:1), and the filtrate was distilled under reduced pressure. Afterwards, the resulting product was washed with ethyl acetate, filtered under reduced pressure, and dried under reduced pressure, so as to obtain 95 g (yield: 91 %) of the title compound.

¹H-NMR (300MHz, DMSO-*d*₆) δ 9.11-9.10 (m, 1H), 8.67~8.66 (m, 1H), 8.29~8.26 (m, 1H), 8.00 (s, 1H), 7.58-7.54 (m, 1H), 3.95-3.93 (m, 4H), 3.80~3.78 (m, 4H).

MS (ESI⁺) *m*/*z* 333.05 [M+H]⁺.

### Process 4) Preparation of 4-(2-(3-nitrophenyl)-6-(pyridin-4-yl)thieno[3,2-d]pyrimidin-4-yl)morpholin

95 g (285 mmol) of the compound prepared in Process 3), 71.4 g (427.5 mmol) of 3-nitrophenyl boronic acid, 23.1 g (20 mmol) of tetrakis(triphenylphosphine)palladium, and 118.2 g (855 mmol) of potassium carbonate were diluted in 1.33 L of a mixed solution containing dioxane and water (at a volume ratio of 3:1), and then stirred at 100 °C for 16 hours. After completion of the reaction, the resulting reaction mixture was cooled to room temperature and distilled under reduced pressure. The solid thus obtained was washed with ethanol and ethyl acetate in the stated order and filtered under reduced pressure, and the filtrate was dried under reduced pressure, so as to obtain 101 g (yield: 84 %) of the title compound.

¹H-NMR (300MHz, DMSO-*d*₆) δ 9.17-9.16 (m, 2H), 8.87-8.85 (m, 1H), 8.69-8.67 (m, 1H), 8.39-8.34 (s, 2H), 8.23 (s, 1H), 7.86-7.60 (m, 1H), 7.60-7.56 (m, 1H), 4.09-4,07 (m, 4H), 3.87-3.86 (m, 4H).

MS (ESI⁺) *m*/*z* 420.11 [M+H]⁺.

### Process 5) Preparation of 3-(4-morpholino-6-(pyridin-4-yl)thieno[3,2-d]pyrimidin-2-yl)aniline

100 g (238 mmol) of the compound prepared in Process 4) was diluted in 2.4 mL of a mixed solvent containing ethanol and distilled water (at a volume ratio of 3:1), and 4.46 g (71.4 mmol) of zinc and 7.62 g (142.8 mmol) of ammonium chloride were added thereto, followed by stirring at 100 °C for 1 hour. After completion of the reaction, the resulting reaction mixture was filtered through a filter filled with Celite to remove the zinc therefrom, and the filtrate was distilled under reduced pressure. The residue thus obtained was diluted with ethyl acetate and washed with a saturated aqueous solution of sodium bicarbonate. The organic layer thus obtained was dried with anhydrous sodium sulfate and filtered under reduced pressure, and the filtrate was dried under reduced pressure, so as to obtain 65 g (yield: 70 %) of the title compound.

¹H-NMR (300MHz, DMSO-*d*₆) δ 9.13~9.12 (m, 1H), 8.66~6.64 (m, 1H), 8.32~8.23 (m, 1H), 8.05 (s, 1H), 7.69~7.68 (m, 1H), 7.61 (s, 1H), 7.59~7.54 (m, 2H), 7.17~7.11 (m, 1H), 6.71~6.68 (m, 1H), 5.20~5.15 (m, 2H), 4.04~4,01 (m, 4H), 3.84~3.81 (m, 4H).

MS (ESI⁺) *m*/*z* 390.13 [M+H]⁺.

### Process 6) Preparation of N-(3-(4-morpholino-6-(pyridin-4-yl)thieno[3,2-d]pyrimidin-2-yl)phenyl)isonicotinamide

41 mg (0.105 mmol) of the compound prepared in Process 5) was diluted in 1 mL of *N,N*-dimethylformamide 1, and 26 mg (0.21 mmol) of isonicotinic acid, 160 mg (1.42 mmol) of (1-[bis(dimethylamino)methylene]-1*H*-1,2,3-triazolo[4,5-b]pyridinium 3-oxide hexafluorophosphate, and 92 µL (0.53 mmol) of diisopropylethylamine were added thereto, followed by stirring at room temperature for 3 hours. After completion of the reaction, 10 mL of water was added, and the produced solid was filtered. The product thus obtained was then washed with ethyl acetate and filtered under reduced pressure, and the filtrate was dried under reduced pressure, so as to obtain 34 mg (yield: 65 %) of the title compound.

¹H-NMR (300MHz, DMSO-*d*₆) δ 10.64 (bs, 1H), 8.79~8.77 (m, 3H), 8.69~8.67 (m, 2H), 8.22 (s, 1H), 8.19~8.17 (m, 1H), 7.96~7.93 (m, 1H), 7.90~7.85 (m, 4H), 7.51~7.45 (t, 1H), 4.05~4.02 (m, 4H), 3.83~3.80 (m, 4H).

MS (ESI⁺) *m*/*z* 495.15 [M+H]⁺.

### Example 2: Preparation of N-(3-(4-morpholino-6-(pyridin-3-yl)thieno[3,2-d]pyrimidin-2-yl)phenyl)isonicotinamide

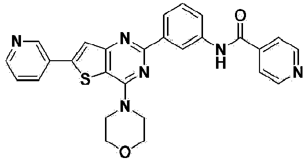

72 mg (yield: 57 %) of the title compound was obtained in the same manner as in Example 1, except that 3-pyridine boronic acid was used instead of the 4-pyridine boronic acid in Process 3) in Example 1.

¹H-NMR (300MHz, DMSO-*d*₆) δ 10.65 (bs, 1H), 9.15 (s, 1H), 8.81~8.79 (m, 3H), 8.66~8.64 (m, 1H), 8.31~8.27 (m, 1H), 8.22~8.18 (m, 1H), 8.10 (s, 1H), 7.99~7.96 (m, 1H), 7.92~7.90 (m, 2H), 7.57~7.53 (m, 1H), 7.52~7.47 (m, 1H), 4.07~4.04 (m, 4H), 3.85~3.81 (m, 4H).

MS (ESI⁺) *m*/*z* 495.15 [M+H]⁺.

### Example 3: Preparation of N-(3-(4-morpholino-6-(oxazol-5-yl)thieno[3,2-d]pyrimidin-2-yl)phenyl)isonicotinamide

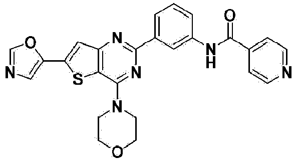

37 mg (yield: 42 %) of the title compound was obtained in the same manner as in Example 1, except that 5-oxazole boronic acid was used instead of the 4-pyridine boronic acid in Process 3) in Example 1.

¹H-NMR (300MHz, DMSO-*d*₆) δ 10.67 (s, 1H), 8.82~8.78 (m, 3H), 8.62 (s, 1H), 8.22~8.19 (d, 1H), 8.10 (s, 1H), 8.01~7.98 (d, 1H), 7.94~7.90 (m, 3H), 7.88 (s, 1H), 7.54~7.48 (t, 1H), 4.06~4.03 (m, 4H), 3.85~3.82 (m, 4H).

MS (ESI⁺) *m*/*z* 485.13 [M+H]⁺.

### Example 4: Preparation of N-(3-(4-morpholino-6-(pyridin-4-yl)thieno[3,2-d]pyrimidin-2-yl)phenyl)nicotinamide

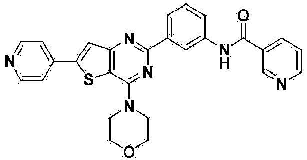

83 mg (yield: 65 %) of the title compound was obtained in the same manner as in Example 1, except that nicotinic acid was used instead of the isonicotinic acid in Process 6) in Example 1.

¹H-NMR (300MHz, DMSO-*d*₆) δ 10.59 (bs, 1H), 9.15~9.14 (m, 1H), 8.79~8.75 (m, 2H), 8.71~8.69 (d, 2H), 8.35~8.31 (m, 1H), 8.24 (s, 1H), 8.20~8.17 (m, 1H), 7.98~7.95 (m, 1H), 7.89~7.87 (d, 2H), 7.60~7.56 (m, 1H), 7.51~7.46 (t, 1H), 4.07~4.04 (m, 4H), 3.84~3.81 (m, 4H).

MS (ESI⁺) *m*/*z* 495.15 [M+H]⁺.

### Example 5: Preparation of N-(3-(4-morpholino-6-(pyridin-3-yl)thieno[3,2-d]pyrimidin-2-yl)phenyl)nicotinamide

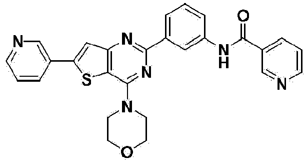

71 mg (yield: 55 %) of the title compound was obtained in the same manner as in Example 1, except that 3-pyridine boronic acid was used instead of the 4-pyridine boronic acid in Process 3) in Example 1 and nicotinic acid was used instead of the isonicotinic acid in Process 6) in Example 1.

¹H-NMR (300MHz, DMSO-*d*₆) δ 10.67 (s, 1H), 8.82~8.78 (m, 3H), 8.62 (s, 1H), 8.22~8.19 (d, 1H), 8.10 (s, 1H), 7.98~8.01 (d, 1H), 7.94~7.90 (m, 3H), 7.88 (s, 1H), 7.54~7.48 (t, 1H), 4.06~4.03 (m, 4H), 3.85~3.82 (m, 4H).

MS (ESI⁺) *m*/*z* 495.15 [M+H]⁺.

### Example 6: Preparation of N-(3-(6-(4-aminophenyl)-4-morpholinothieno[3,2-d]pyrimidin-2-yl)phenyl)nicotinamide hydrochloride

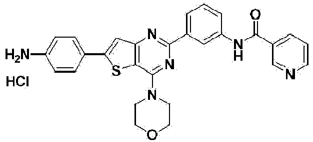

23 mg (yield: 32 %) of the title compound was obtained in the same manner as in Example 1, except that 4-(t-butylcarbonyl)-aminophenyl boronic acid was used instead of the 4-pyridine boronic acid in Process 3) in Example 1 and nicotinic acid was used instead of the isonicotinic acid in Process 6) in Example 1.

¹H-NMR (300MHz, DMSO-*d*₆) δ 10.68 (s, 1H), 9.19, 9.18 (d, 1H), 8.82~8.80 (m, 1H), 8.76 (s, 1H), 8.42~8.38 (m, 1H), 8.17, 8.15 (d, 1H), 8.02, 8.00 (d, 1H), 7.67~7.62 (m, 4H), 7.57, 7.55 (d, 1H), 6.74, 6.71 (d, 2H), 4.08 (m, 4H), 3.85 (m, 4H).

MS (ESI⁺) *m*/*z* 508.92 [M+H]⁺.

### Example 7: Preparation of N-(3-(4-morpholino-6-(oxazol-5-yl)thieno[3,2-d]pyrimidin-2-yl)phenyl)nicotinamide

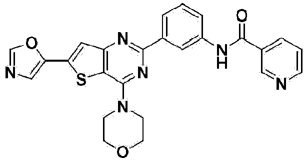

82 mg (yield: 77 %) of the title compound was obtained in the same manner as in Example 1, except that 5-oxazole boronic acid was used instead of the 4-pyridine boronic acid in Process 3) in Example 1 and nicotinic acid was used instead of the isonicotinic acid in Process 6) in Example 1.

¹H-NMR (300MHz, DMSO-*d*₆) δ 10.61 (s, 1H), 9.16~9.15 (m, 1H), 8.79~8.77 (m, 2H), 8.62 (s, 1H), 8.37~8.33 (m, 1H), 8.21~8.18 (d, 1H), 8.01~7.98 (m, 1H), 7.94 (s, 1H), 7.88 (s, 1H), 7.61~7.59 (m, 1H), 7.57~7.48 (t, 1H), 4.06~4.03 (m, 4H), 3.85~3.82 (m, 4H).

MS (ESI⁺) *m*/*z* 485.13 [M+H]⁺.

### Example 8: Preparation of N-(3-(4-morpholino-6-(1H-pyrazole-4-yl)thieno[3,2-d]pyrimidin-2-yl)phenyl) isonicotinamide

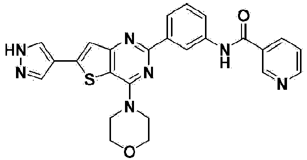

21 mg (yield: 16 %) of the title compound was obtained in the same manner as in Example 1, except that 1-(t-butylcarbonyl)-pyrazole-4-boronic acid was used instead of the 4-pyridine boronic acid in Process 3) in Example 1 and nicotinic acid was used instead of the isonicotinic acid in Process 6) in Example 1.

¹H-NMR (300MHz, DMSO-*d*₆) δ 13.24 (s, 1H), 10.60 (s, 1H), 9.16 (s, 1H), 8.78~8.77 (m, 2H), 8.36 (d, 1H), 8.20~8.17 (m, 2H), 7.98 (d, 1H), 7.66 (s, 1H), 7.61~7.57 (m, 1H), 7.52~7.47 (m, 2H), 4.07~4.04 (m, 4H), 3.85~3.81 (m, 4H).

MS (ESI⁺) *m*/*z* 484.55 [M+H]⁺.

### Example 9: Preparation of N-(3-(4-morpholino-6-(1H-pyrrolo[2,3-b]pyridin-4-yl)thieno[3,2-d]pyrimidin-2-yl)phenyl)nicotinamide

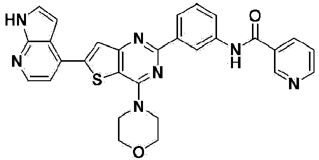

71 mg (yield: 81 %) of the title compound was obtained in the same manner as in Example 1, except that 1-(t-butylcarbonyl)-7-azaindole-4-boronic acid pinacol ester was used instead of the 4-pyridine boronic acid in Process 3) in Example 1 and nicotinic acid was used instead of the isonicotinic acid in Process 6) in Example 1.

¹H-NMR (300MHz, DMSO-*d*₆) δ 10.62 (s, 1H), 9.18 (m, 1H), 8.81 (m, 2H), 8.39 (m, 2H), 8.23 (m, 1H), 8.15 (s, 1H), 8.00 (m, 1H), 7.73 (m, 1H), 7.61 (m, 1H), 7.53 (m, 1H), 7.05 (m, 1H), 4.14 (m, 4H), 3.86 (m, 4H)

MS (ESI⁺) *m*/*z* 534.16 [M+H]⁺.

### Example 10: Preparation of N-(3-(4-morpholino-6-(1H-pyrazolo[3,4-b]pyridin-4-yl)thieno[3,2-d]pyrimidin-2-yl)phenyl)nicotinamide

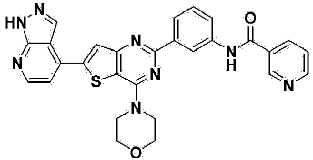

5 mg (yield: 8 %) of the title compound was obtained in the same manner as in Example 1, except that 1-(t-butylcarbonyl)-7-pyrazolo[3,4-b]pyridin-4-boronic acid pinacol ester was used instead of the 4-pyridine boronic acid in Process 3) in Example 1 and nicotinic acid was used instead of the isonicotinic acid in Process 6) in Example 1.

¹H-NMR (300MHz, DMSO-*d*₆) δ 14.04 (s, 1H), 10.64 (s, 1H), 9.17~9.16 (m, 1H), 8.83 (s, 1H), 8.80~8.78 (m, 1H), 8.76 (s, 1H), 8.67~8.66 (m, 1H), 8.38~8.34 (m, 2H), 8.26~8.23 (d, 1H), 8.01~7.98 (m, 1H), 7.76~7.75 (m, 1H), 7.63~7.59 (m, 1H), 7.57~7.48 (t, 1H), 4.14~4.09 (m, 4H), 3.89~3.87 (m, 4H).

MS (ESI⁺) *m*/*z* 535.16 [M+H]⁺.

### Example 11: Preparation of N-(3-(4-morpholino-6-(1H-pyrrolo[2,3-c]pyridin-4-yl)thieno[3,2-d]pyrimidin-2-yl)phenyl)nicotinamide

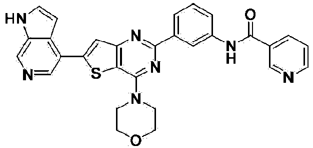

4 mg (yield: 99 %) of the title compound was obtained in the same manner as in Example 1, except that 1-(t-butylcarbonyl)-6- pyrrolo[2,3-c]pyridin-4-boronic acid pinacol ester was used instead of the 4-pyridine boronic acid in Process 3) in Example 1 and nicotinic acid was used instead of the isonicotinic acid in Process 6) in Example 1.

¹H-NMR (300MHz, DMSO-*d*₆) δ 12.11 (s, 1H), 10.65 (s, 1H), 9.17 (m, 1H), 8.85~8.81 (m, 3H), 8.64 (m, 1H), 8.38~8.36 (m, 1H), 8.24~8.22 (m, 1H), 8.02~7.99 (m, 2H), 7.84 (m, 2H), 7.60~7.51 (m, 2H), 7.06 (m, 1H), 4.10 (m, 4H), 3.87 (m, 4H).

MS (ESI⁺) *m*/*z* 534.16 [M+H]⁺.

### Example 12: Preparation of N-(3-(4-morpholino-6-(pyridin-3-yl)thieno[3,2-d]pyrimidin-2-yl)phenyl)oxazole-4-carboxamide

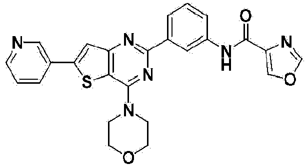

61 mg (yield: 61 %) of the title compound was obtained in the same manner as in Example 1, except that 3-pyridine boronic acid was used instead of the 4-pyridine boronic acid in Process 3) in Example 1 and oxazol-4-carboxylic acid was used instead of the isonicotinic acid in Process 6) in Example 1.

¹H-NMR (300MHz, DMSO-*d*₆) δ 10.30 (bs, 1H), 9.15 (s, 1H), 8.84~8.83 (m, 2H), 8.66~8.63 (m, 2H), 8.31~8.27 (m, 1H), 8.18~8.15 (m, 1H), 8.11 (s, 1H), 7.94~7.91 (m, 1H), 7.57~7.52 (m, 1H), 7.48~7.43 (t, 1H), 4.07~4.04 (m, 4H), 3.85~3.81 (m, 4H).

MS (ESI⁺) *m*/*z* 485.13 [M+H]⁺.

### Example 13: Preparation of N-(3-(4-morpholino-6-(pyridin-3-yl)thieno[3,2-d]pyrimidin-2-yl)phenyl)oxazole-5-carboxamide

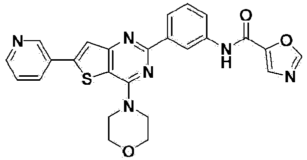

44 mg (yield: 35 %) of the title compound was obtained in the same manner as in Example 1, except that 3-pyridine boronic acid was used instead of the 4-pyridine boronic acid in Process 3) in Example 1 and oxazol-5-carboxylic acid was used instead of the isonicotinic acid in Process 6) in Example 1.

¹H-NMR (300MHz, DMSO-*d*₆) δ 10.61 (s, 1H), 9.17~9.16 (m, 1H), 8.77~8.76(m, 1H), 8.68~8.67 (m, 2H), 8.34~8.31 (m, 1H), 8.23~8.20 (m, 1H), 8.12 (s, 1H), 8.06 (s, 1H), 7.96~7.93 (m, 1H), 7.60~7.56 (m, 1H), 7.54~7.48 (t, 1H), 4.09~4.06 (m, 4H), 3.87~3.84 (m, 4H).

MS (ESI⁺) *m*/*z* 485.13 [M+H]⁺.

### Example 14: Preparation of N-(3-(4-morpholino-6-(pyridin-3-yl)thieno[3,2-d]pyrimidin-2-yl)phenyl)oxazole-2-carboxamide

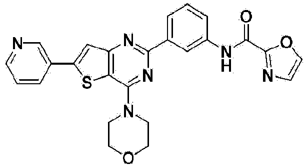

37 mg (yield: 29 %) of the title compound was obtained in the same manner as in Example 1, except that 3-pyridine boronic acid was used instead of the 4-pyridine boronic acid in Process 3) in Example 1 and oxazol-2-carboxylic acid was used instead of the isonicotinic acid in Process 6) in Example 1.

¹H-NMR (300MHz, DMSO-*d*₆) δ 10.32 (s, 1H), 9.16 (s, 1H), 8.87 (s, 1H), 8.85 (s, 1H), 8.66 (m, 1H), 8.65 (s, 1H), 8.32 (d, 1H), 8.19 (d, 1H), 8.14 (s, 1H), 7.95 (d, 1H), 7.57 (q, 1H), 7.48 (t, 1H), 4.07 (t, 4H), 3.87 (t, 4H).

MS (ESI⁺) *m*/*z* 485.13 [M+H]⁺.

### Example 15: Preparation of 2-methyl-N-(3-(4-morpholino-6-(pyridin-3-yl)thieno[3,2-d]pyrimidin-2-yl)phenyl)oxazole-5-carboxamide

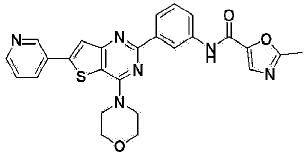

35 mg (yield: 45 %) of the title compound was obtained in the same manner as in Example 1, except that 3-pyridine boronic acid was used instead of the 4-pyridine boronic acid in Process 3) in Example 1 and 2-methyloxazol-5-carboxylic acid was used instead of the isonicotinic acid in Process 6) in Example 1.

¹H-NMR (300MHz, DMSO-*d*₆) δ 10.47 (s, 1H), 9.16 (s, 1H), 8.73 (s, 1H), 8.68 (d, 1H), 8.33~8.29 (m, 1H), 8.19 (d, 1H), 8.18 (s, 1H), 7.93~7.91 (m, 2H), 7.59~7.55 (m, 1H), 7.49 (t, 1H), 4.08~4.05 (m, 4H), 3.86~3.83 (m, 4H), 2.55 (s, 3H).

MS (ESI⁺) *m*/*z* 499.56 [M+H]⁺.

### Example 16: Preparation of 4-methyl-N-(3-(4-morpholino-6-(pyridin-3-yl)thieno[3,2-d]pyrimidin-2-yl)phenyl)oxazole-5-carboxamide

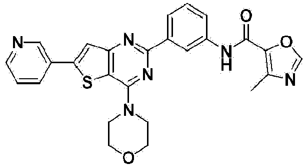

73 mg (yield: 56 %) of the title compound was obtained in the same manner as in Example 1, except that 3-pyridine boronic acid was used instead of the 4-pyridine boronic acid in Process 3) in Example 1 and 4-methyloxazol-5-carboxylic acid was used instead of the isonicotinic acid in Process 6) in Example 1.

¹H-NMR (300MHz, DMSO-*d*₆) δ 10.44 (s, 1H), 9.16 (s, 1H), 8.81 (s, 1H), 8.67 (d, 1H), 8.56 (s, 1H), 8.31 (d, 1H), 8.19 (d, 1H), 8.13 (s, 1H), 7.90 (d, 1H), 7.57 (q, 1H), 7.48 (t, 1H) 4.07 (t, 4H), 3.85 (t, 4H), 2.47 (s, 3H).

MS (ESI⁺) *m*/*z* 499.15 [M+H]⁺.

### Example 17: Preparation of 2,4-dimethyl-N-(3-(4-morpholino-6-(pyridin-3-yl)thieno[3,2-d]pyrimidin-2-yl)phenyl)oxazole-5-carboxamide

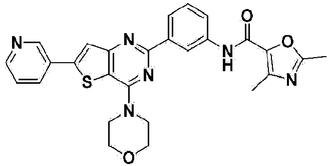

108 mg (yield: 81 %) of the title compound was obtained in the same manner as in Example 1, except that 3-pyridine boronic acid was used instead of the 4-pyridine boronic acid in Process 3) in Example 1 and 2,4-dimethyloxazol-5-carboxylic acid was used instead of the isonicotinic acid in Process 6) in Example 1.

¹H-NMR (300MHz, DMSO-*d*₆) δ 10.30 (s, 1H), 9.16 (s, 1H), 8.80 (s, 1H), 8.67 (d, 1H), 8.32 (d, 1H), 8.19 (d, 1H), 8.14 (s, 1H), 7.89 (d, 1H), 7.59 (q, 1H), 7.47 (t, 1H), 4.09 (t, 4H), 3.85 (t, 4H), 2.49 (s, 3H), 2.41 (s, 3H).

MS (ESI⁺) *m*/*z* 513.16 [M+H]⁺.

### Example 18: Preparation of N-(3-(4-morpholino-6-(pyridin-3-yl)thieno[3,2-d]pyrimidin-2-yl)phenyl)thiazole-4-carboxamide

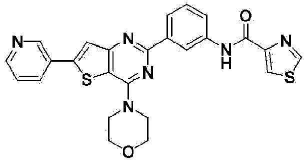

63 mg (yield: 49 %) of the title compound was obtained in the same manner as in Example 1, except that 3-pyridine boronic acid was used instead of the 4-pyridine boronic acid in Process 3) in Example 1 and thiazole-4-carboxylic acid was used instead of the isonicotinic acid in Process 6) in Example 1.

¹H-NMR (300MHz, DMSO-*d*₆) δ 10.46 (bs, 1H), 9.28 (s, 1H), 9.13 (bs, 1H), 8.90 (s, 1H), 8.65~8.63 (m, 1H), 8.53~8.52 (m, 1H), 8.31~8.27 (m, 1H), 8.18~8.15 (m, 1H), 8.12 (s, 1H), 7.97~7.94 (m, 1H), 7.56~7.52 (m, 1H), 7.48'7.43 (m, 1H), 4.07~4.04 (m, 4H), 3.84~3.81 (m, 4H).

MS (ESI⁺) *m*/*z* 501.11 [M+H]⁺.

### Example 19: Preparation of N-(3-(4-morpholino-6-(pyridin-3-yl)thieno[3,2-d]pyrimidin-2-yl)phenyl)thiazole-5-carboxamide

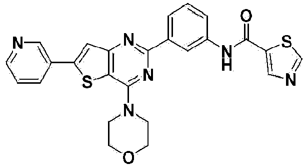

43 mg (yield: 34 %) of the title compound was obtained in the same manner as in Example 1, except that 3-pyridine boronic acid was used instead of the 4-pyridine boronic acid in Process 3) in Example 1 and thiazole-5-carboxylic acid was used instead of the isonicotinic acid in Process 6) in Example 1.

¹H-NMR (300MHz, DMSO-*d*₆) δ 10.62 (bs, 1H), 9.31 (s, 1H), 9.14~9.13 (m, 1H), 8.76 (s, 1H), 8.73~8.71 (m, 1H), 8.66~8.64 (m, 1H), 8.32~8.28 (m, 1H), 8.20~8.18 (m, 1H), 8.09 (s, 1H), 7.94~7.91 (m, 1H), 7.57~7.52 (m, 1H), 7.49~7.46 (m, 1H), 4.07~4.04 (m, 4H), 3.85~3.82 (m, 4H).

MS (ESI⁺) m/z 501.11 [M+H]⁺.

### Example 20: Preparation of N-(3-(4-morpholino-6-(pyridin-3-yl)thieno[3,2-d]pyrimidin-2-yl)phenyl)thiazole-2-carboxamide

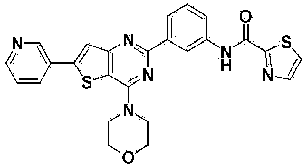

80 mg (yield: 63 %) of the title compound was obtained in the same manner as in Example 1, except that 3-pyridine boronic acid was used instead of the 4-pyridine boronic acid in Process 3) in Example 1 and thiazole-2-carboxylic acid was used instead of the isonicotinic acid in Process 6) in Example 1.

¹H-NMR (300MHz, DMSO-*d*₆) δ 10.90 (bs, 1H), 9.13 (s, 1H), 8.93~8.92 (m, 1H), 8.65~8.63 (m, 1H), 8.31~8.27 (m, 1H), 8.21~8.12 (m, 3H), 7.98~7.95 (m, 1H), 7.57~7.52 (m, 1H), 7.50~7.45 (t, 1H), 4.07~4.04 (m, 4H), 3.85~3.82 (m, 4H).

MS (ESI⁺) m/z 501.11 [M+H]⁺.

### Example 21: Preparation of 2-methyl-N-(3-(4-morpholino-6-(pyridin-3-yl)thieno[3,2-d]pyrimidin-2-yl)phenyl)thiazole-5-carboxamide

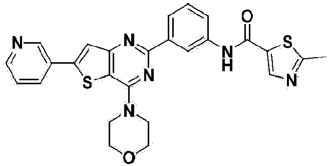

96 mg (yield: 60 %) of the title compound was obtained in the same manner as in Example 1, except that 3-pyridine boronic acid was used instead of the 4-pyridine boronic acid in Process 3) in Example 1 and 2-methylthiazole-5-carboxylic acid was used instead of the isonicotinic acid in Process 6) in Example 1.

¹H-NMR (300MHz, DMSO-*d*₆) δ 10.53 (s, 1H), 9.17 (m, 1H), 8.72 (m, 1H), 8.67 (m, 1H), 8.52 (s, 1H), 8.35 (m, 1H), 8.21 (m, 1H), 8.11 (s, 1H), 7.91 (m, 1H), 7.56 (m, 1H), 7.48 (m, 1H), 4.08 (m, 4H), 3.86 (m, 4H), 2.73 (s, 3H).

MS (ESI⁺) *m*/*z* 515.12 [M+H]⁺.

### Example 22: Preparation of 4-methyl-N-(3-(4-morpholino-6-(pyridin-3-yl)thieno[3,2-d]pyrimidin-2-yl)phenyl)thiazole-5-carboxamide

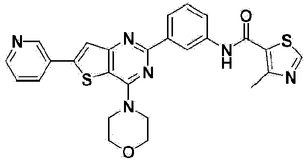

64 mg (yield: 48 %) of the title compound was obtained in the same manner as in Example 1, except that 3-pyridine boronic acid was used instead of the 4-pyridine boronic acid in Process 3) in Example 1 and 4-methylthiazole-5-carboxylic acid was used instead of the isonicotinic acid in Process 6) in Example 1.

¹H-NMR (300MHz, DMSO-*d*₆) δ 10.39 (s, 1H), 9.15 (m, 1H), 8.72~8.71 (m, 1H), 8.67~8.65 (m, 1H), 8.33~8.29 (m, 1H), 8.21~8.18 (m, 1H), 8.12 (s, 1H), 7.88~7.85 (m, 1H), 7.59~7.56 (m, 1H), 7.51 (t, 1H), 4.08~4.05 (m, 4H), 3.86~3.84 (m, 4H).

MS (ESI⁺) *m*/*z* 515.12 [M+H]⁺.

### Example 23: Preparation of 2,4-dimethyl-N-(3-(4-morpholino-6-(pyridin-3-yl)thieno[3,2-d]pyrimidin-2-yl)phenyl)thiazole-5-carboxamide

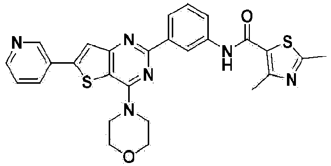

118 mg (yield: 74 %) of the title compound was obtained in the same manner as in Example 1, except that 3-pyridine boronic acid was used instead of the 4-pyridine boronic acid in Process 3) in Example 1 and 2,4-dimethylthiazole-5-carboxylic acid was used instead of the isonicotinic acid in Process 6) in Example 1.

¹H-NMR (300MHz, DMSO-*d*₆) δ 10.26 (s, 1H), 9.17 (m, 1H), 8.72 (m, 1H), 8.67 (m, 1H), 8.34 (m, 1H), 8.21 (m, 1H), 8.10 (s, 1H), 7.84 (m, 1H), 7.58 (m, 1H), 7.45 (m, 1H), 4.07 (m, 4H), 3.85 (m, 4H), 2.69 (s, 3H), 2.58 (s, 3H).

MS (ESI⁺) *m*/*z* 529.14 [M+H]⁺.

### Example 24: Preparation of N-(3-(4-morpholino-6-(pyridin-3-yl)thieno[3,2-d]pyrimidin-2-yl)phenyl)piperidine-3-carboxamide

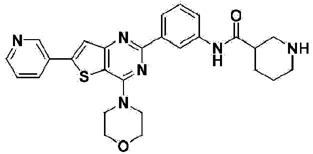

140 mg (yield: 52 %) of the title compound was obtained in the same manner as in Example 1, except that 3-pyridine boronic acid was used instead of the 4-pyridine boronic acid in Process 3) in Example 1 and 1-(t-butylcarbonyl)piperidine-3-carboxylic acid was used instead of the isonicotinic acid in Process 6) in Example 1.

¹H-NMR (300MHz, DMSO-*d*₆) δ 10.11 (s, 1H), 9.14 (d, 1H), 8.67 (dd, 1H), 8.60 (s, 1H), 8.32 (m, 1H), 8.10 (s, 1H), 8.09 (d, 1H), 7.82 (m, 1H), 7.58 (m, 1H), 7.43 (t, 1H), 4.46 (m, 1H), 4.06 (m, 4H), 3.85 (m, 4H), 3.11 (m, 1H), 2.89 (m, 1H), 2.66 (m, 1H), 1.92 (m, 1H), 1.66 (m, 2H), 1.55 (m, 1H), 1.39 (m, 1H), 0.82 (m, 1H).

MS (ESI⁺) *m*/*z* 501.12 [M+H]⁺.

### Example 25: Preparation of N-(3-(4-morpholino-6-(pyridin-3-yl)thieno[3,2-d]pyrimidin-2-yl)phenyl)piperidine-4-carboxamide

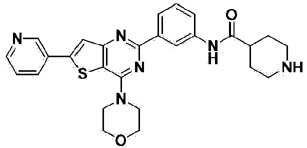

1.8 mg (yield: 94 %) of the title compound was obtained in the same manner as in Example 1, except that 3-pyridine boronic acid was used instead of the 4-pyridine boronic acid in Process 3) in Example 1 and 1-(t-butylcarbonyl)piperidine-4-carboxylic acid was used instead of the isonicotinic acid in Process 6) in Example 1.

¹H-NMR (300MHz, DMSO-*d*₆) δ 10.19 (s, 1H), 9.16~9.15 (m, 1H), 8.68~8.66 (m, 1H), 8.62 (m, 1H), 8.33`8.29 (m, 1H), 8.14~8.10 (m, 2H), 7.84~7.81 (m, 1H), 7.59~7.55 (m, 1H), 7.46~7.40 (t, 1H), 4.08~4.04 (m, 4H), 3.86~3.83 (m, 4H), 2.96~2.89 (m, 2H), 2.73~2.64 (m, 1H), 1.99~1.80 (m, 5H).

MS (ESI⁺) *m*/*z* 501.20 [M+H]⁺.

### Example 26: Preparation of 1-(2-hydroxyethyl)-N-(3-(4-morpholino-6-(pyridin-3-yl)thieno[3,2-d]pyrimidin-2-yl)phenyl)piperidine-4-carboxamide

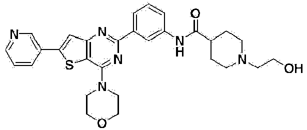

7 mg (yield: 23 %) of the title compound was obtained in the same manner as in Example 1, except that 3-pyridine boronic acid was used instead of the 4-pyridine boronic acid in Process 3) in Example 1 and 1-(2-hydroxyethyl)piperidine-4-carboxylic acid was used instead of the isonicotinic acid in Process 6) in Example 1.

¹H-NMR (300MHz, DMSO-*d*₆) δ 9.14 (m, 1H), 8.67~8.65 (m, 1H), 8.43~8.40 (m, 1H), 8.33~8.28 (m, 2H), 8.16 (s, 1H), 7.62~7.55 (m, 2H), 7.48 (m, 1H), 4.70 (brs, 1H), 4.06~4.03 (m, 4H), 3.85~3.81 (m, 4H), 3.72 (m, 2H), 3.49 (m, 2H), 2.90-2.86 (m, 2H), 2.32~2.18 (m, 2H), 1.57~1.54 (m, 4H).

MS (ESI⁺) *m*/*z* 545.23 [M+H]⁺.

### Example 27: Preparation of 1-(2-methoxyethyl)-N-(3-(4-morpholino-6-(pyridin-3-yl)thieno[3,2-d]pyrimidin-2-yl)phenyl)piperidine-4-carboxamide

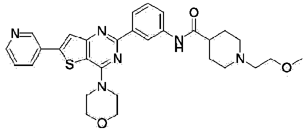

25 mg (yield: 22 %) of the title compound was obtained in the same manner as in Example 1, except that 3-pyridine boronic acid was used instead of the 4-pyridine boronic acid in Process 3) in Example 1 and 1-(2-methoxyethyl)piperidine-4-carboxylic acid was used instead of the isonicotinic acid in Process 6) in Example 1.

¹H-NMR (300MHz, DMSO-*d*₆) δ 10.01 (s, 1H), 9.15 (s, 1H), 8.66 (d, 1H), 8.65 (s, 1H), 8.32 (d, 1H), 8.15 (s, 1H), 8.10 (s, 1H), 7.82 (d, 1H), 7.58~7.54 (m, 1H), 7.40 (t, 1H), 4.07~4.04 (m, 4H), 3.85~3.81 (m, 4H), 3.45~3.39 (m, 4H), 3.32 (s, 3H), 2.98~2.94 (m, 2H), 2.35~2.31 (m 1H), 2.08~2.02 (m, 2H), 1.99~1.77 (m, 2H), 1.69~1.61 (m, 2H).

MS (ESI⁺) *m*/*z* 559.70 [M+H]⁺.

### Example 28: Preparation of 1-formyl-N-(3-(4-morpholino-6-(pyridin-3-yl)thieno[3,2-d]pyrimidin-2-yl)phenyl)piperidine-4-carboxamide

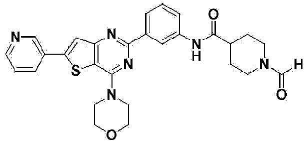

4 mg (yield: 3 %) of the title compound was obtained in the same manner as in Example 1, except that 3-pyridine boronic acid was used instead of the 4-pyridine boronic acid in Process 3) in Example 1 and 1-formylpiperidine-4-carboxylic acid was used instead of the isonicotinic acid in Process 6) in Example 1.

¹H-NMR (300MHz, DMSO-*d*₆) δ 10.12 (s, 1H), 9.16 (d, 1H), 8.67 (dd, 1H), 8.62 (s, 1H), 8.34~8.30 (m, 1H), 8.13~8.10 (m, 2H), 8.03 (s, 1H), 7.86~7.83 (m, 1H), 7.51 (dd, 1H), 7.43 (d, 1H), 4.23 (m, 1H), 4.07 (m, 4H), 3.85 (m, 4H), 3.77 (m, 1H), 3.10 (m, 2H), 2.74~2.64 (m, 2H), 1.91~1.86 (m, 2H), 1.60~1.44 (m, 2H).

MS (ESI⁺) *m*/*z* 529.19 [M+H]⁺.

### Example 29: Preparation of 1-acetyl-N-(3-(4-morpholino-6-(pyridin-3-yl)thieno[3,2-d]pyrimidin-2-yl)phenyl)piperidine-4-carboxamide

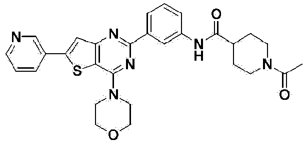

70 mg (yield: 65 %) of the title compound was obtained in the same manner as in Example 1, except that 3-pyridine boronic acid was used instead of the 4-pyridine boronic acid in Process 3) in Example 1 and 1-acetylpiperidine-4-carboxylic acid was used instead of the isonicotinic acid in Process 6) in Example 1.

¹H-NMR (300MHz, DMSO-*d*₆) δ 10.09 (s, 1H), 9.16, 9.15 (d, 1H), 8.68~8.67 (m, 1H), 8.67 (d, 1H), 8.34~8.30 (m, 1H), 8.13~8.10 (m, 2H), 7.85 (d, 1H), 7.60~7.56 (m, 1H), 7.42 (t, 1H), 4.08~4.05 (m, 4H), 3.87~3.85 (m, 4H), 3.62 (s, 3H), 3.18 (d, 1H), 2.93~2.88 (m, 2H), 1.89~1.82 (m, 2H), 1.61~1.53 (m, 2H), 1.26~1.24 (m, 1H), 0.96~0.84 (m, 1H).

MS (ESI⁺) *m*/*z* 559.4 [M+H]⁺.

### Example 30: Preparation of methyl 4-((3-(4-morpholino-6-(pyridin-3-yl)thieno[3,2-d]pyrimidin-2-yl)phenyl)carbamoyl)piperidine-1 -carboxylate

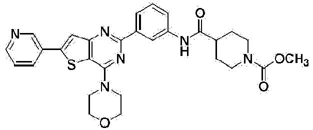

54 mg (yield: 48 %) of the title compound was obtained in the same manner as in Example 1, except that 3-pyridine boronic acid was used instead of the 4-pyridine boronic acid in Process 3) in Example 1 and 1-(methoxycarbonyl)piperidine-4-carboxylic acid was used instead of the isonicotinic acid in Process 6) in Example 1.

¹H-NMR (300MHz, DMSO-*d*₆) δ 10.08 (s, 1H), 9.14 (d, 1H), 8.67 (dd, 1H), 8.61 (s, 1H), 8.32 (m, 1H), 8.11 (m, 1H), 8.10 (s, 1H), 7.84 (d, 1H), 7.58 (m, 1H), 7.44 (t, 1H), 4.44 (d, 1H), 4.05 (m, 4H), 3.85 (m, 1H), 3.84 (m, 4H), 3.12 (t, 1H), 2.64 (m, 2H), 2.02 (s, 3H), 1.90 (m, 2H), 1.55 (m, 2H).

MS (ESI⁺) *m*/*z* 543.30 [M+H]⁺.

### Example 31: Preparation of ethyl 4-((3-(4-morpholino-6-(pyridin-3-yl)thieno[3,2-d]pyrimidin-2-yl)phenyl)carbamoyl)piperidine-1-carboxylate

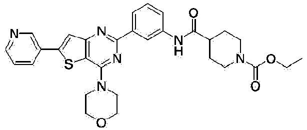

14 mg (yield: 12 %) of the title compound was obtained in the same manner as in Example 1, except that 3-pyridine boronic acid was used instead of the 4-pyridine boronic acid in Process 3) in Example 1 and 1-(ethoxycarbonyl)piperidine-4-carboxylic acid was used instead of the isonicotinic acid in Process 6) in Example 1.

¹H-NMR (300MHz, DMSO-*d*₆) δ 10.09 (s, 1H), 9.15~9.14 (m, 1H), 8.68~8.65 (m, 1H), 8.62~8.60 (m, 1H), 8.33~8.29 (m, 1H), 8.12~8.09 (m, 2H), 7.85~7.82 (m, 2H), 7.59~7.54 (m, 1H), 7.44~7.39 (t, 1H), 4.09~4.02 (m, 6H), 3.86~3.83 (m, 4H), 2.90~2.85 (m, 2H), 2.73~2.50 (m, 2H), 1.85~1.81 (m, 2H), 1.59~1.47 (m, 2H), 1.22~1.17 (t, 3H).

MS (ESI⁺) *m*/*z* 573.22 [M+H]⁺.

### Example 32: Preparation of (1s,4s)-4-hydroxy-N-(3-(4-morpholino-6-(pyridin-3-yl)thieno[3,2-d]pyrimidin-2-yl)phenyl)cyclohexane-1 -carboxamide

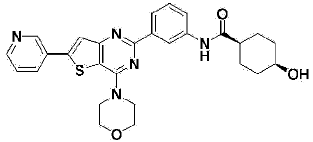

112 mg (yield: 84 %) of the title compound was obtained in the same manner as in Example 1, except that 3-pyridine boronic acid was used instead of the 4-pyridine boronic acid in Process 3) in Example 1 and (1s,4s)-4-hydroxycyclohexylcarboxylic acid was used instead of the isonicotinic acid in Process 6) in Example 1.

¹H-NMR (300MHz, DMSO-*d*₆) δ 9.94 (s, 1H), 9.16 (d, 1H), 8.68~8.66 (m, 1H), 8.63 (m, 1H), 8.34~8.30 (m, 1H), 8.12~8.10 (m, 2H), 7.86~7.83 (m, 1H), 7.60~7.56 (m, 1H), 7.44~7.39 (m, 1H), 4.36~4.35 (m, 1H), 4.08~4.05 (m, 4H), 3.87~3.84 (m, 5H), 2.40 (m, 1H), 1.92~1.84 (m, 2H), 1.75~1.71 (m, 2H), 1.57~1.45 (m, 4H).

MS (ESI⁺) *m*/*z* 516.2 [M+H]⁺.

### Example 33: Preparation of N-(3-(4-morpholino-6-(oxazol-5-yl)thieno[3,2-d]pyrimidin-2-yl)phenyl)oxazole-4-carboxamide

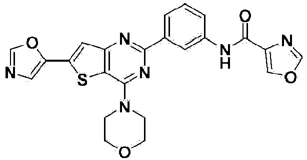

80 mg (yield: 77 %) of the title compound was obtained in the same manner as in Example 1, except that 5-oxazole boronic acid was used instead of the 4-pyridine boronic acid in Process 3) in Example 1 and 4-oxazole carboxylic acid was used instead of the isonicotinic acid in Process 6) in Example 1.

¹H-NMR (300MHz, DMSO-*d*₆) δ 10.31 (s, 1H), 8.84 (m, 1H), 8.64~8.62 (m, 2H), 8.18~8.15 (d, 1H), 7.98 (m, 1H), 7.94 (s, 1H), 7. 88 (s, 1H), 7.50~7.45 (t, 1H), 4.05 (m, 4H), 3.85~3.84 (m, 4H).

MS (ESI⁺) *m*/*z* 475.11 [M+H]⁺.

### Experimental Example : PlKfyve enzyme inhibition assay

For each of the compounds prepared in Examples 1 to 33, the PlKfyve enzyme inhibitory activity was measured by using a QS S Assis PIKFYVE (PIP5K3)_ADP-Glo^{™} Kit (Cat. #11-118) made by Carna Bioscience. 5 µL of each of human-derived PlKfyve kinase, PI(3)P, and ATP solution was added to a 384-well plate (such that final concentrations of each solution are 375 ng/mL, 10 µm, and 2 µm, respectively). The compound to be evaluated underwent serial dilution according to the set concentrations, and 5 µL of the diluted solution was added to each well and caused a reaction for 60 minutes. MgCl₂ was mixed in ADP-Glo^{™} solution (Promega, Cat. #V9102) to a final concentration of 100 mM, and 20 µL of the mixed solution was added to each well and caused a reaction at room temperature for 40 minutes. 40 µL of kinase detection solution was added to each well and caused a reaction at room temperature for 40 minutes. Then, the luminescence signals were detected by using a Synergy^{™} NEO microplate analyzer. Well to which no enzyme was added were used as a negative control, whereas wells to which no drug was added were used as a positive control, so as to calculate the enzyme inhibitory ability (%) of drugs. The 50 % inhibitor concentration (IC₅₀) values were calculated by using a GraphPad Prism software.

Results thereof are shown in Table 1. In Table 1, when the case where the IC₅₀ value was 50 nM or less was indicated as A, the case where the IC₅₀ value was greater than 50 nM but equal to or less than 100 nM was indicated as B, and the case where the IC₅₀ value was greater than 100 nM was indicated as C.

**[Table1] PlKfyve enzyme inhibitory assay**

| Example | IC₅₀ (nM) | Example | IC₅₀ (nM) | Example | IC₅₀ (nM) |
|---|---|---|---|---|---|
| 1 | A | 12 | A | 23 | B |
| 2 | A | 13 | A | 24 | A |
| 3 | A | 14 | A | 25 | A |
| 4 | A | 15 | B | 26 | B |
| 5 | A | 16 | A | 27 | B |
| 6 | A | 17 | A | 28 | C |
| 7 | A | 18 | C | 29 | C |
| 8 | A | 19 | A | 30 | C |
| 9 | A | 20 | C | 31 | A |
| 10 | B | 21 | A | 32 | A |
| 11 | C | 22 | A | 33 | A |

### Industrial Applicability

The present disclosure provides a thieno[3,2-d]pyrimidine derivative compound which is useful as a PlKfyve kinase inhibitor, a pharmaceutically acceptable salt thereof, a preparation method thereof, and an intermediate thereof.

Hereinabove, the present disclosure was examined with respect to the specific embodiments. Those of ordinary skill in the art to which the present disclosure pertains will understand that the present disclosure may be implemented in a modified form without departing from the essential characteristics of the present disclosure. Therefore, the disclosed specific embodiments are to be considered in an descriptive point of view rather than a restrictive sense. The scope of the present disclosure is indicated in the claims rather than the above description, and differences within the scope equivalent thereto should be construed as being included in the invention.

## Claims

1. A compound Formula (1) or a pharmaceutically acceptable salt thereof: 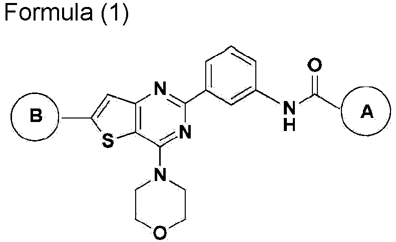 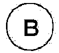
is 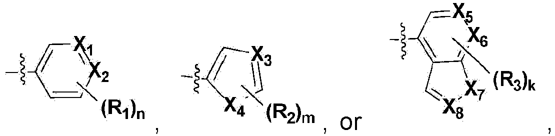 wherein X₁, X₂, X₃, X₅, X₆, or X₃ are each independently CH or a nitrogen atom, and
X₄ and X₇ are each independently CH₂, an oxygen atom, or NH,
in each case, R₁, R₂, and R₃ are each independently one selected from a halogen, an amino group, -NH(C₁-C₃alkyl), -N(C₁-C₃alkyl)₂, C₁-C₆alkyl, C₂-C₆alkenyl, and C₁-C₆alkoxy,
n is an integer of 0 to 4,
m is an integer from 0 to 2,
k is an integer of 0 to 4, 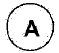
is 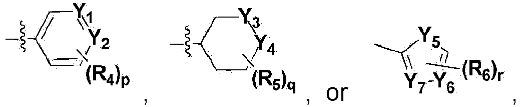 wherein Y₁, Y₂, Y₆, and Y₇ are each independently CH or a nitrogen atom, and
Y₃, Y₄, and Y₅ are each independently CH₂, an oxygen atom, NH, or a sulfur atom,
in each case, R₄ and R₆ are each independently one selected from hydrogen, a halogen, an amino group, C₁-C₆alkyl, C₂-C₆alkenyl, and C₁-C₆alkoxy,
R₅ is one selected from hydrogen, -T-OH, -T-CHO, -T-(C₁-C₄alkyl), and -T-(C₁-C₄alkoxy), wherein -T- is absent or is -C(=O), C₁-C₄alkylene or C₂-C₄alkenylene,
p is an integer of 0 to 4,
q is an integer of 0 to 4, and
r is an integer from 0 to 2.

2. The compound of claim 1, wherein 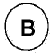 is one of

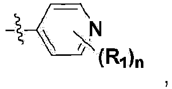

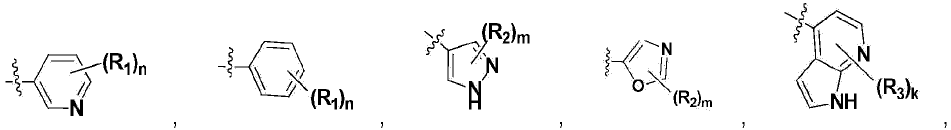

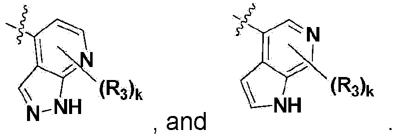

3. The compound of claim 1, wherein 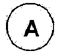 is one of

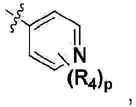

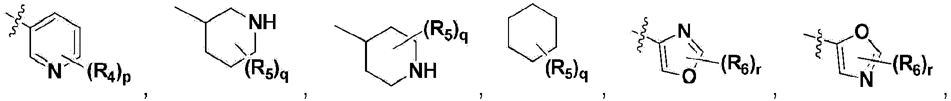

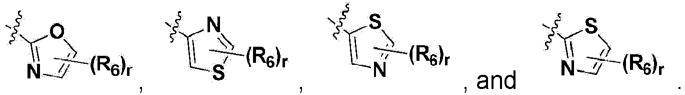

4. The compound of claim 1, wherein R₁ is a halogen or an amino group.

5. The compound of claim 1, wherein R₅ is one of hydroxy, -(C₁-C₄alkylene)-OH, -(C₁-C₄alkylene)-(C₁-C₄alkoxy), -C(=O)H, -C(=O)(C₁-C₄alkyl), -C(=O)-(C₁-C₄alkoxy), and -(C₁-C₄alkylene)-CHO.

6. The compound of claim 1, wherein R₆ is one of methyl, ethyl, and propyl.

7. The compound of claim 1, wherein the compound is one selected from the group consisting of the following compounds.
| | **Structural Formula** | **Name** |
|---|---|---|
| **1** | 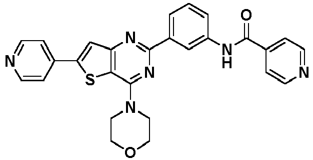 | *N*-(3-(4-morpholino-6-(pyridin-4-yl)thieno[3,2-*d*]pyrimidin-2-yl)phenyl)isonicotinamide |
| **2** | 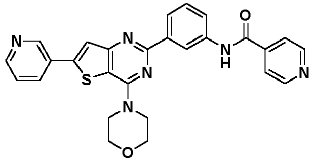 | *N*-(3-(4-morpholino-6-(pyridin-3-yl)thieno[3,2-*d*]pyrimidin-2-yl)phenyl)isonicotinamide |
| **3** | 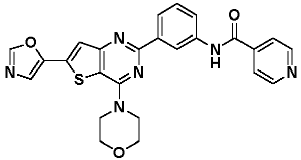 | *N*-(3-(4-morpholino-6-(oxazol-5-yl)thieno[3,2-d]pyrimidin-2-yl)phenyl)isonicotinamide |
| **4** | 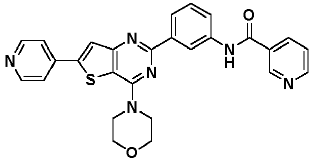 | *N*-(3-(4-morpholino-6-(pyridin-4-yl)thieno[3,2-*d*]pyrimidin-2-yl)phenyl)nicotinamide |
| **5** | 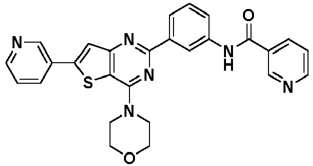 | *N*-(3-(4-morpholino-6-(pyridin-3-yl)thieno[3,2-*d*]pyrimidin-2-yl)phenyl)nicotinamide |
| **6** | 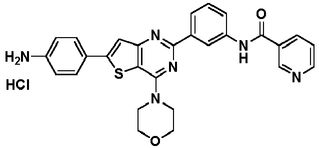 | *N*-(3-(6-(4-aminophenyl)-4-morpholinothieno[3,2-*d*]pyrimidin-2-yl)phenyl)nicotinamide hydrochloride |
| **7** | 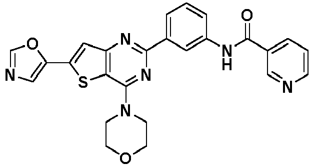 | *N*-(3-(4-morpholino-6-(oxazol-5-yl)thieno[3,2-*d*]pyrimidin-2-yl)phenyl)nicotinamide |
| **8** | 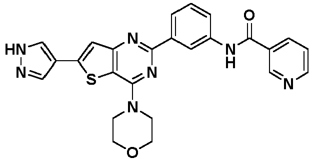 | *N*-(3-(4-morpholino-6-(1H-pyrazole-4-yl)thieno[3,2-*d*]pyrimidin-2-yl)phenyl)isonicotinamide |
| **9** | 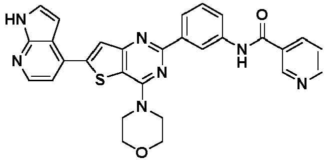 | *N*-(3-(4-morpholino-6-(1H-pyrrolo[2,3-*b*]pyridin-4-yl)thieno[3,2-*d*]pyrimidin-2-yl)phenyl)nicotinamide |
| **10** | 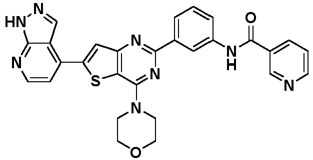 | *N*-(3-(4-morpholino-6-(1H-pyrazolo[3,4-*b*]pyridin-4-yl)thieno[3,2-*d*]pyrimidin-2-yl)phenyl)nicotinamide |
| **11** | 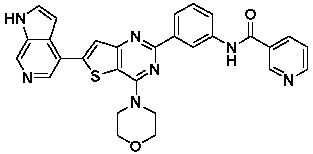 | *N*-(3-(4-morpholino-6-(1H-pyrrolo[2,3-c]pyridin-4-yl)thieno[3,2-*d*]pyrimidin-2-yl)phenyl)nicotinamide |
| **12** | 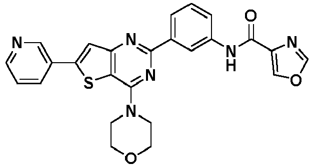 | *N*-(3-(4-morpholino-6-(pyridin-3-yl)thieno[3,2-*d*]pyrimidin-2-yl)phenyl)oxazole-4-carboxamide |
| **13** | 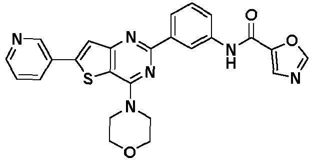 | *N*-(3-(4-morpholino-6-(pyridin-3-yl)thieno[3,2-*d*]pyrimidin-2-yl)phenyl)oxazole-5-carboxamide |
| **14** | 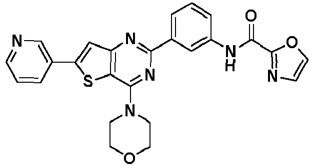 | *N*-(3-(4-morpholino-6-(pyridin-3-yl)thieno[3,2-*d*]pyrimidin-2-yl)phenyl)oxazole-2-carboxamide |
| **15** | 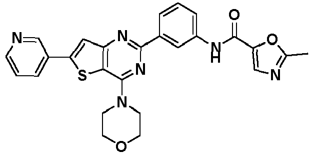 | 2-methyl-*N*-(3-(4-morpholino-6-(pyridin-3-yl)thieno[3,2-*d*]pyrimidin-2-yl)phenyl)oxazole-5-carboxamide |
| **16** | 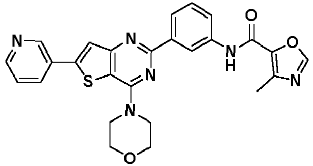 | 4-methyl-*N*-(3-(4-morpholino-6-(pyridin-3-yl)thieno[3,2-*d*]pyrimidin-2-yl)phenyl)oxazole-5-carboxamide |
| **17** | 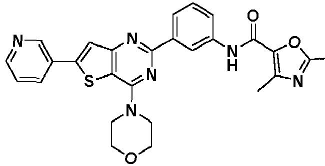 | 2,4-dimethyl-*N*-(3-(4-morpholino-6-(pyridin-3-yl)thieno[3,2-*d*]pyrimidin-2-yl)phenyl)oxazole-5-carboxamide |
| **18** | 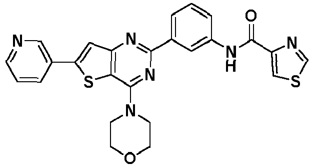 | *N*-(3-(4-morpholino-6-(pyridin-3-yl)thieno[3,2-*d*]pyrimidin-2-yl)phenyl)thiazole-4-carboxamide |
| **19** | 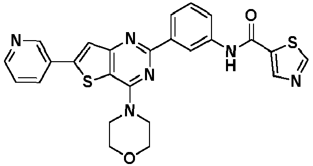 | *N*-(3-(4-morpholino-6-(pyridin-3-yl)thieno[3,2-*d*]pyrimidin-2-yl)phenyl)thiazole-5-carboxamide |
| **20** | 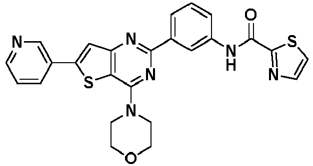 | *N*-(3-(4-morpholino-6-(pyridin-3-yl)thieno[3,2-*d*]pyrimidin-2-yl)phenyl)thiazole-2-carboxamide |
| **21** | 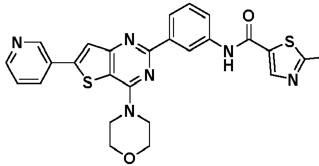 | 2-methyl-*N*-(3-(4-morpholino-6-(pyridin-3-yl)thieno[3,2-*d*]pyrimidin-2-yl)phenyl)thiazole-5-carboxamide |
| **22** | 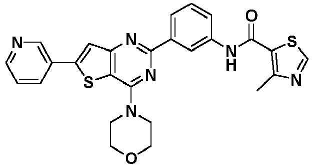 | 4-methyl-*N*-(3-(4-morpholino-6-(pyridin-3-yl)thieno[3,2-*d*]pyrimidin-2-yl)phenyl)thiazole-5-carboxamide |
| **23** | 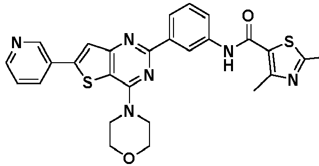 | 2,4-dimethyl-*N*-(3-(4-morpholino-6-(pyridin-3-yl)thieno[3,2-*d*]pyrimidin-2-yl)phenyl)thiazole-5-carboxamide |
| **24** | 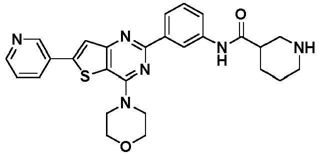 | *N*-(3-(4-morpholino-6-(pyridin-3-yl)thieno[3,2-*d*]pyrimidin-2-yl)phenyl)piperidine-3-carboxamide |
| **25** | 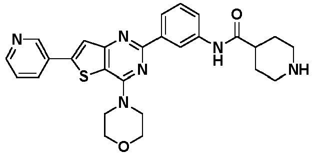 | *N*-(3-(4-morpholino-6-(pyridin-3-yl)thieno[3,2-*d*]pyrimidin-2-yl)phenyl)piperidine-4-carboxamide |
| **26** | 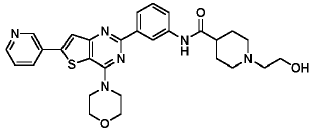 | 1-(2-hydroxyethyl)-*N*-(3-(4-morpholino-6-(pyridin-3-yl)thieno[3,2-*d*]pyrimidin-2-yl)phenyl)piperidine-4-carboxamide |
| **27** | 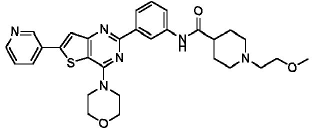 | 1-(2-methoxyethyl)-*N*-(3-(4-morpholino-6-(pyridin-3-yl)thieno[3,2-*d*]pyrimidin-2-yl)phenyl)piperidine-4-carboxamide |
| **28** | 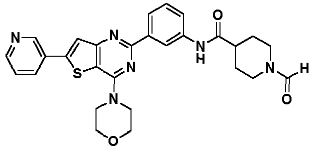 | 1-formyl-*N*-(3-(4-morpholino-6-(pyridin-3-yl)thieno[3,2-*d*]pyrimidin-2-yl)phenyl)piperidine-4-carboxamide |
| **29** | 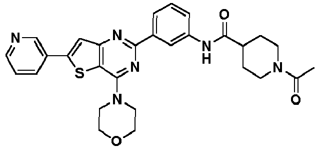 | 1-acetyl-*N*-(3-(4-morpholino-6-(pyridin-3-yl)thieno[3,2-*d*]pyrimidin-2-yl)phenyl)piperidine-4-carboxamide |
| **30** | 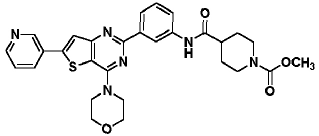 | methyl 4-((3-(4-morpholino-6-(pyridin-3-yl)thieno[3,2-*d*]pyrimidin-2-yl)phenyl)carbamoyl)piperidine-1-carboxylate |
| **31** | 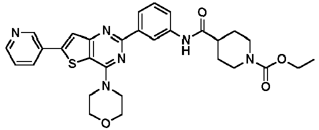 | ethyl 4-((3-(4-morpholino-6-(pyridin-3-yl)thieno[3,2-*d*]pyrimidin-2-yl)phenyl)carbamoyl)piperidine-1-carboxylate |
| **32** | 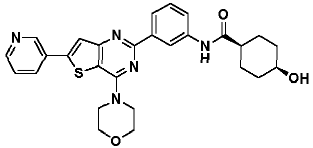 | (1s,4s)-4-hydroxy-*N*-(3-(4-morpholino-6-(pyridin-3-yl)thieno[3,2-*d*]pyrimidin-2-yl)phenyl)cyclohexane-1-carboxamide |
| **33** | 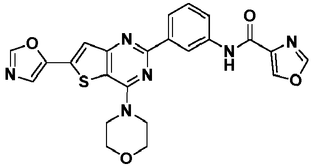 | *N*-(3-(4-morpholino-6-(oxazol-5-yl)thieno[3,2-*d*]pyrimidin-2-yl)phenyl)oxazole-4-carboxamide |
